# EUROPÄISCHE PATENTSCHRIFT

(11) **EP 3 003 408 B1**
(45) Veröffentlichungstag und Bekanntmachung des Hinweises auf die Patenterteilung: **24.05.2017**
(21) Anmeldenummer: 14729615.6
(22) Anmeldetag: 26.05.2014
(51) Int. Cl.: A61L 9/22, F24F 3/16, H01T 23/00

(54) **LUFTIONISATIONSMODUL**
AIR IONIZATION MODULE
MODULE D'IONISATION DE L'AIR

(30) Priorität: 29.05.2013 DE 102013210114
(43) Veröffentlichungstag der Anmeldung: 13.04.2016
(73) Patentinhaber: LK Luftqualität AG, 6014 Luzern (CH)
(72) Erfinder: WEIBEL, Beda, CH-6430 Schwyz (CH); OSWALD, Michael, CH-6003 Luzern (CH)
(74) Vertreter: Kailuweit & Uhlemann Patentanwälte Partnerschaft mbB
(86) Internationale Anmeldenummer: PCT/EP2014/060812
(87) Internationale Veröffentlichungsnummer: WO 2014/191346

(56) Entgegenhaltungen:
- EP-A1- 1 348 448
- WO-A1-2005/029924
- GB-A- 1 177 891
- US-A- 4 188 530

## Beschreibung

Die Erfindung betrifft Luftionisationsmodule mit lösbar in Fassungen angeordneten Ionisationsröhren und einen Träger mit den Fassungen.

Bekannt ist, dass mit Ionisierungsapparaten die Raum- und damit die Atemluft behandelt werden kann. Dabei werden Bakterien und andere Keime abgetötet und Großmoleküle in kleinmolekulare Fragmente aufgespalten. Komplexe und große Moleküle sind unter anderem Geruchsstoffe, so dass mit einer Luftionisation eine Geruchsbelastung unterdrückt werden kann. Weiterhin können auch Mikroorganismen in der Luft wirksam reduziert werden.

In Ionisationsapparaten werden elektrische Felder zwischen zwei Elektroden mit Spannungspotenzialen ausgenutzt, um durch Gasentladungen Ionen durch Stoßionisationen zu erzeugen. Dazu werden bekannterweise Ionisierungsröhren in Form von Glasröhren mit einer Innenelektrode und einer Außenelektrode eingesetzt, wobei ein koaxialer Aufbau vorhanden ist. Wird eine für die Gasentladung ausreichend hohe elektrische Spannung angelegt, bildet das Glas der Wandung ein Dielektrikum, in dem ein großes elektrisches Feld vorhanden ist. Die durchströmende Luft wird mit Ionen angereichert. Nachteilig ist dabei, dass es ab einer bestimmten Spannung zu einer Bildung von Ozon kommt, die sich mit der Vergrößerung der Spannung erhöht.

US4188530 ofenbart Luftionisationsvorrichtungen mit mehreren Ionisationsröhren, wobei mehrere Röhren auf Platten angebracht sind. Die Zuführung der Elektrizität erfolgt direkt an Lagern durch Kabel.

Durch die Druckschrift DE 43 34 956 C2 sind ein Verfahren zur Luftbehandlung mit Ionen und eine Vorrichtung zur Durchführung des Verfahrens bekannt, wobei die Langzeitstabilität eines Ionisationsapparates erhöht wird. Hauptaugenmerk gilt der Vermeidung einer erhöhten Ozonerzeugung. Dazu werden Sensoren in Form eines Luftqualitätssensors, eines Luftströmungsfühlers und eines Luftfeuchtefühlers eingesetzt. Beim Auftreten sowohl äußerer Störquellen wie zum Beispiel bei Smog, einer Inversionswetterlage, Gewitter, äußere Energiefelder als auch innerer Störquellen durch den Betrieb elektrischer Geräte kann die Belastung an Ozon in der Zuluft in unerwünschtem Maß ansteigen und zu einer Grenzwertüberschreitung führen.

In der Druckschrift DE 100 07 523 C2 wird ein Verfahren zur Luftbehandlung mit Ionen sowie eine Vorrichtung zur Durchführung des Verfahrens beschrieben, wobei zusätzlich ein Ozonsensor zur Bestimmung des Ozongehaltes eingesetzt wird.

Auf die Problematik einer Kondensation von Wasser hervorgerufen durch einen Temperaturunterschied zwischen Umgebungsluft und beispielsweise gekühlter Zuluft wird in diesen Druckschriften nicht eingegangen.

Der im Patentanspruch 1 angegebenen Erfindung liegt die Aufgabe zugrunde, ein kompaktes Luftionisationsmodul zur Anreicherung eines Luftstromes mit Ionen weitestgehend ohne Kondensation zu schaffen.

Diese Aufgabe wird mit den im Patentanspruch 1 aufgeführten Merkmalen gelöst.

Die Luftionisationsmodule mit lösbar in Fassungen angeordneten Ionisationsröhren und einen Träger mit den Fassungen zeichnen sich insbesondere dadurch aus, dass weitestgehend keine Kondensation bei einer Anreicherung eines Luftstromes mit Ionen vorhanden ist.

Dazu weist der Träger zwei beabstandet zueinander angeordnete Platten auf, wobei eine erste Platte eine Montageplatte und eine zweite Platte eine Leiterplatte mit den Fassungen sind. Weiterhin befindet sich zwischen den Platten ein Körper aus einem wärmeisolierenden Material, so dass die zweite Platte gegenüber der ersten Platte wärmeisoliert angeordnet ist.

Das Luftionisationsmodul dient der Erzeugung von Ionen in einem Luftstrom für wenigstens einen Innenraum eines Gebäudes. Ionisierte Luft führt beispielsweise zu einer Spaltung vieler geruchsbildender Moleküle, zur Abtötung von Mikroorganismen, zum Abbau gasförmiger flüchtiger Kohlenwasserstoffe und zur Absenkung des Oxidpotentials der Luft. Dadurch wird eine behagliche naturnahe Luft im damit beaufschlagten Raum geschaffen.

Die dabei verwendeten Ionisationsröhren dienen der Erzeugung der Ionen durch Koronaentladungen. Das erfolgt mittels an den Ionisationsröhren anliegender hoher elektrischer Wechselspannung. Dazu besitzt eine bekannte Ionisationsröhre beispielsweise eine Anode und eine gitterförmige Kathode, die die Anode ummantelt. Die Kathode ist dabei vorteilhafterweise geerdet.

Das Luftionisationsmodul besitzt einen Träger aus einer Montageplatte und einer Leiterplatte, die gegeneinander wärmeisolierend angeordnet sind. Bekannterweise besteht die Leiterplatte aus einem elektrischen Isolator auf dem Leiterbahnen und eventuell Kontaktstellen aus einem elektrisch leitenden Material aufgebracht sind. Der Körper aus einem wärmeisolierenden Material befindet sich zwischen diesen beiden Platten. Damit besteht vorteilhafterweise keine Wärmebrücke zwischen diesen beiden Platten. Weiterhin ist das Material insbesondere auch nicht brennbar, so dass auch Schwelbrände vermeidbar sind. Der Körper kann dazu vorteilhafterweise aus einem Kunststoff, insbesondere einem Polyisocyanurat (PIR) oder einem Polyurethan (PU) bestehen. Das Luftionisationsmodul ist bekannterweise ein Bestandteil des Zuluftkanals. Dabei kann der Träger gleichzeitig ein Bestandteil einer Wand des Zuluftkanals sein. Die Montage und Demontage vereinfacht sich wesentlich. Das ist bei Reinigungs- und Wartungsarbeiten insbesondere der Ionisationsröhren besonders vorteilhaft. Die Anordnung in der Wand des Zuluftkanals ist platzsparend. Darüber hinaus kann sich wenigstens ein Luftionisationsmodul im Zuluftkanal oder in der raumlufttechnischen Anlage selbst befinden. Darüber hinaus kann sich wenigstens ein Luftionisationsmodul im Zuluftkanal befinden. Die Ionisationsröhren ragen in den Kanalraum und damit in den Zuluftstrom. Bei Zuführung gekühlter Zuluft bei ansonsten vorherrschenden höheren Temperaturen wird mit Einsatz eines Luftionisationsmoduls mit fehlender Wärmebrücke im Träger eine Kondensation von Wasser vermieden. Eine ansonsten aus dem kondensierten Wasser und den mit Hochspannung betriebenen Ionisationsröhren resultierende Gefährdung wird vermieden. Damit kann das Luftionisationsmodul auch bei höheren Lufttemperaturen und relativen Luftfeuchtigkeiten sicher betrieben werden. Die Lebensdauer des Moduls wird dadurch erhöht.

Vorteilhafte Ausgestaltungen der Erfindung sind in den Patentansprüchen 2 bis 11 angegeben.

Der Körper aus dem wärmeisolierenden Material befindet sich nach der Weiterbildung des Patentanspruchs 2 zwischen den Platten und einer diesen Körper ummantelnden Wand aus einem wenigstens bereichsweise wärmeisolierenden Material. Damit bilden die Platten und die Wand eine kompakte Einheit. Der Körper kann dabei aus den unterschiedlichsten Materialien bestehen, wobei das auch ein aus einem elastischen Material bestehender Körper sein kann.

Die Platten sind nach der Weiterbildung des Patentanspruchs 3 über Rastmechanismen miteinander verbunden, wobei Rastelemente in Aussparungen eingreifen. Damit ist eine leichte Montage aber auch Demontage gegeben.

Die Montageplatte ist nach der Weiterbildung des Patentanspruchs 4 größer als die Leiterplatte. Damit kann das Luftionisationsmodul leicht in eine Wand eines Kanals ohne Ausbilden einer Wärmebrücke integriert werden. Das kann über eine beabstandete Anordnung zwischen Leiterplatte und Kanalwand erfolgen. Natürlich kann auch ein eine Lücke zwischen Leiterplatte und Kanalwand überbrückender Körper aus einem nicht wärmeleitenden Material angeordnet sein.

Die Fassungen befinden sich nach der Weiterbildung des Patentanspruchs 5 in Körper aus dem wärmeisolierenden Material, so dass diese bündig mit der nach außen weisenden Oberfläche der Leiterplatte sind.

Nach der Weiterbildung des Patentanspruchs 6 sind die Fassungen mit Leiterbahnen der Leiterplatte elektrisch verbunden und damit gleichzeitig befestigt. Die Anoden der Ionisationsröhren sind über Fassungen und Leiterbahnen der Leiterplatte miteinander verbunden. Wenigstens ein Bestandteil eines elektrischen Verbindungselements für eine lösbare Verbindung wenigstens einer Kathode mit mindestens einer Leiterbahn der Leiterplatte ist elektrisch leitend verbunden und damit gleichzeitig befestigt. Die miteinander verbundenen Anoden und Kathoden von Ionisationsröhren sind eine Gruppe von Ionisationsröhren.

Die Anoden der Ionisationsröhren einer Gruppe sind nach der Weiterbildung des Patentanspruchs 7 mit der Sekundärwicklung eines Transformators zusammengeschaltet. Die Primärwicklung des Transformators ist über einen Schalter mit einem elektrischen Niederspannungsnetz verbindbar. Darüber hinaus sind die Kathoden der Ionisationsröhren geerdet.

Die Betätigungseinrichtung eines mechanisch wirkenden oder die Steuerelektrode eines elektrisch steuerbaren Schalters ist nach der Weiterbildung des Patentanspruchs 8 mit einem Datenverarbeitungssystem verbunden, so dass mittels des Datenverarbeitungssystems ein automatischer und/oder vorgegebener Betrieb der Ionisationsröhren gegeben ist.

Transformatoren für Gruppen von Ionisationsröhren sind nach der Weiterbildung des Patentanspruchs 9 in einem Gehäuse angeordnet. Das Gehäuse ist weiterhin an der Montageplatte befestigt, so dass die Ionisationsröhren, der Träger und das Gehäuse ein kompaktes Luftionisationsmodul sind.

Der Körper aus dem wärmeisolierenden Material zwischen der Montageplatte und der Leiterplatte besteht nach der Weiterbildung des Patentanspruchs 10 aus einem geschäumten Kunststoff. Der Kunststoff wird dazu einfach in den Raum zwischen diese Platten eingebracht und bildet sich beim Aushärten selbst aus, wobei auch mit Luft befüllte Blasen entstehen, so dass eine gute Wärmeisolation zwischen den Platten vorhanden ist. Gleichzeitig kann der vorhandene Raum einfach vollständig ausgefüllt werden.

Günstigerweise sind nach der Weiterbildung des Patentanspruchs 11 vier Ionisationsröhren eine Gruppe von Ionisationsröhren. Diese sind weiterhin vorteilhafterweise an den Eckpunkten eines keinen rechten Winkel aufweisenden Parallelogramms angeordnet. Damit sind Ionisationsröhren versetzt zueinander angeordnet, so dass eine optimale Luftumströmung der Ionisationsröhren vorhanden ist.

Ein Ausführungsbeispiel der Erfindung ist in den Zeichnungen jeweils prinzipiell dargestellt und wird im Folgenden näher beschrieben.

Es zeigen:
- Fig. 1: ein Luftionisationsmodul in einer Seitenansicht,
- Fig. 2: ein Luftionisationsmodul in einer Draufsicht,
- Fig. 3: einen Träger mit Ionisationsröhren und einem Gehäuse für Transformatoren und
- Fig. 4: eine Schaltung mit Ionisationsröhren.

Ein Luftionisationsmodul besteht im Wesentlichen aus einem Träger 1 mit Ionisationsröhren 2 und einem Gehäuse 3 für Transformatoren 8.

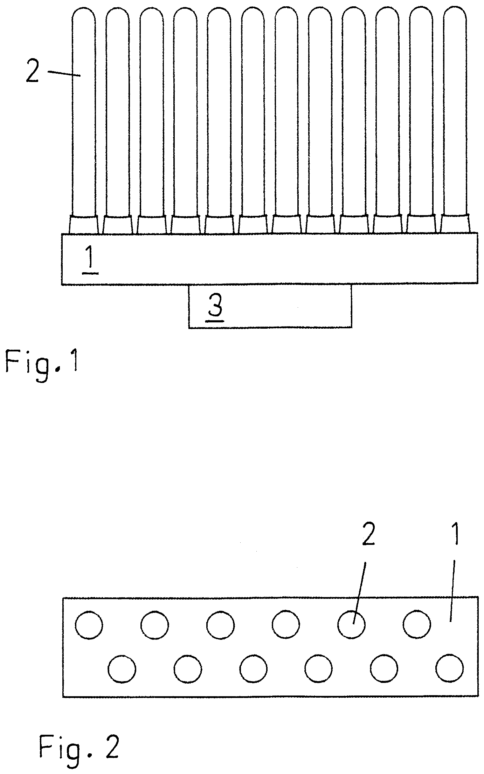

Dazu zeigen
die Fig. 1 ein Luftionisationsmodul in einer prinzipiellen Seitenansicht und
die Fig. 2 ein Luftionisationsmodul in einer prinzipiellen Draufsicht.

Der Träger 1 weist zwei beabstandet zueinander angeordnete Platten 4, 5 auf, wobei eine erste Platte 4 eine Montageplatte 4 und eine zweite Platte 5 eine Leiterplatte 5 sind. Zwischen den Platten 4, 5 befindet sich ein Körper 6 aus einem wärmeisolierenden Material, so dass die zweite Platte 5 gegenüber der ersten Platte 4 wärmeisoliert angeordnet ist. Die Montageplatte 4 ist größer als die Leiterplatte 5 ausgebildet. In Ausführungsformen kann die Montageplatte 4 und/oder die Leiterplatte 5 auch eine U-Form aufweisen. Der Körper 6 aus dem wärmeisolierenden Material besteht aus einem geschäumten Kunststoff. Die Montageplatte 4 ist eine Metallplatte 4 während die Leiterplatte 5 bekannterweise eine Platte 5 aus einem elektrischen Isolator mit aufgebrachten Leiterbahnen ist.

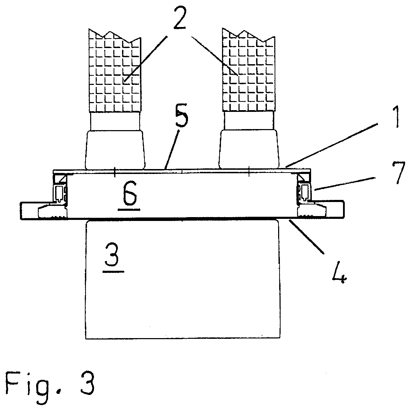

Die Fig. 3 zeigt dazu einen Träger 1 mit Ionisationsröhren 2 und einem Gehäuse 3 für Transformatoren in einer prinzipiellen Darstellung.

Der Körper 6 aus dem wärmeisolierenden Material befindet sich zwischen den Platten 4, 5 und einer diesen Körper 6 ummantelnden Wand 7. Letztere besteht dabei wenigstens bereichsweise aus einem wärmeisolierenden Material, vorzugsweise einem Kunststoff. Die Wand 7 weist vorteilhafterweise gleichzeitig Rastmechanismen auf, wobei Rastelemente in Aussparungen eingreifen.

Die Ionisationsröhren 2 sind lösbar in Fassungen angeordnet.

Die Leiterplatte 5 weist die Fassungen auf, wobei die Anoden der Ionisationsröhren 2 über die Fassungen und Leiterbahnen der Leiterplatte 5 miteinander verbunden sind. Gleichzeitig sind Verbindungselemente für die Kathoden der Ionisationsröhren 2 an oder in der Leiterplatte 5 angeordnet, so dass die Kathoden der Ionisationsröhren 2 über Verbindungselemente und Leiterbahnen miteinander verbunden sind. Dazu sind die Kathoden über Kabel mit den Verbindungselementen mittels Schraubenverbindungen lösbar verbunden. Die miteinander verbundenen Anoden und Kathoden von Ionisationsröhren 2 sind zu einer Gruppe von ionisationsröhren 2 zusammengeschaltet. Eine Gruppe von Ionisationsröhren 2 kann dazu beispielsweise aus vier Ionisationsröhren 2 bestehen, die an den Eckpunkten eines keinen rechten Winkel aufweisenden Parallelogramms angeordnet sind.

Die Fassungen sind mit der Leiterplatte 5 so verbunden, dass diese bündig mit der nach außen weisenden Oberfläche der Leiterplatte 5 sind.

Die Anoden der Ionisationsröhren 2 einer Gruppe sind mit der Sekundärwicklung eines Transformators 8 zusammengeschaltet. Die Primärwicklung des Transformators 8 ist über einen Schalter 9 mit einem elektrischen Niederspannungsnetz 10 verbindbar. Die Kathoden der Ionisationsröhren 2 sind geerdet.

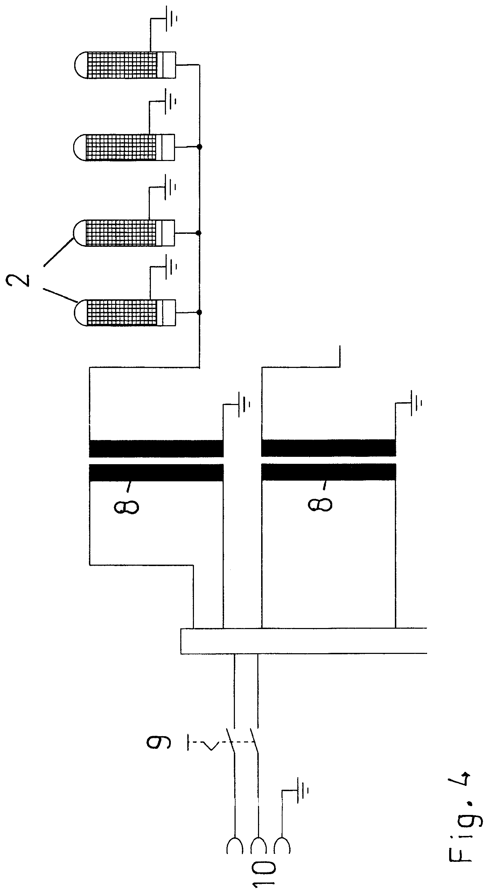

Die Fig. 4 zeigt eine Schaltung mit Ionisationsröhren 2 in einer prinzipiellen Darstellung.

Die Transformatoren 8 für die Gruppen von Ionisationsröhren 2 befinden sich in dem Gehäuse 3, das weiterhin an der Montageplatte 4 befestigt ist. Die Ionisationsröhren 2, der Träger 1 und das Gehäuse 3 mit den Transformatoren 8 ist damit ein kompaktes Luftionisationsmodul.

Die Betätigungseinrichtung eines mechanisch wirkenden oder die Steuerelektrode eines elektrisch steuerbaren Schalters 9 sind mit einem Datenverarbeitungssystem verbunden, so dass ein automatischer und/oder vorgegebener Betrieb der Ionisationsröhren 2 gegeben ist.

## Patentansprüche

1. Luftionisationsmodul mit lösbar in Fassungen angeordneten Ionisationsröhren (2) und einem Träger (1) mit den Fassungen, **dadurch gekennzeichnet, dass** der Träger (1) zwei beabstandet zueinander angeordnete Platten (4, 5) aufweist, wobei eine erste Platte (4) eine Montage-platte (4) und eine zweite Platte (5) eine Leiterplatte (5) mit den Fassungen sind, und dass sich zwischen den Platten (4, 5) ein Körper (6) aus einem wärmeisolierenden Material, bevorzugt aus einem geschäumten Kunststoff, befindet, so dass die zweite Platte (5) gegenüber der ersten Platte (4) wärmeisoliert angeordnet ist.

2. Luftionisationsmodul nach Patentanspruch 1, **dadurch gekennzeichnet, dass** sich der Körper (6) aus dem wärmeisolierenden Material zwischen den Platten (4, 5) und einer diesen Körper (6) ummantelnden Wand (7) aus einem wenigstens bereichsweise wärmeisolierenden Material befindet.

3. Luftionisationsmodul nach Patentanspruch 1, **dadurch gekennzeichnet, dass** die Platten (4, 5) über Rastmechanismen miteinander verbunden sind, wobei Rastelemente in Aussparungen eingreifen.

4. Luftionisationsmodul nach Patentanspruch 1, **dadurch gekennzeichnet, dass** die Montageplatte (4) größer als die Leiterplatte (5) ist.

5. Luftionisationsmodul nach Patentanspruch 1, **dadurch gekennzeichnet, dass** sich die Fassungen im Körper (6) aus dem wärmeisolierenden Material befinden, so dass diese bündig mit der nach außen weisenden Oberfläche der Leiterplatte (5) sind.

6. Luftionisationsmodul nach Patentanspruch 1 oder 5, **dadurch gekennzeichnet, dass** die Fassungen mit Leiterbahnen der Leiterplatte (5) elektrisch verbunden und damit gleichzeitig befestigt sind, dass Anoden der Ionisationsröhren (2) über Fassungen und Leiterbahnen der Leiterplatte (5) miteinander verbunden sind, dass wenigstens ein Bestandteil eines elektrischen Verbindungselements für eine lösbare Verbindung wenigstens einer Kathode mit mindestens einer Leiterbahn der Leiterplatte (5) elektrisch leitend verbunden und damit gleichzeitig befestigt ist und dass die miteinander verbundenen Anoden und Kathoden von Ionisationsröhren (2) eine Gruppe von Ionisationsröhren (2) sind.

7. Luftionisationsmodul nach Patentanspruch 1 oder 6, **dadurch gekennzeichnet, dass** die Anoden der Ionisationsröhren (2) einer Gruppe mit der Sekundärwicklung eines Transformators (8) zusammengeschaltet sind, dass die Primärwicklung des Transformators (8) über einen Schalter (9) mit einem elektrischen Niederspannungsnetz (10) verbindbar ist und dass die Kathoden der Ionisationsröhren (2) geerdet sind.

8. Luftionisationsmodul nach Patentanspruch 7, **dadurch gekennzeichnet, dass** die Betätigungseinrichtung eines mechanisch wirkenden oder die Steuerelektrode eines elektrisch steuerbaren Schalters (9) mit einem Datenverarbeitungssystem verbunden ist, so dass mittels des Datenverarbeitungssystems ein automatischer und/oder vorgegebener Betrieb der Ionisationsröhren (2) gegeben ist.

9. Luftionisationsmodul nach Patentanspruch 1, 6 oder 7, **dadurch gekennzeichnet, dass** Transformatoren (8) für Gruppen von Ionisationsröhren (2) in einem Gehäuse (3) angeordnet sind und dass das Gehäuse (3) an der Montageplatte (4) befestigt ist, so dass die Ionisationsröhren (2), der Träger (1) und das Gehäuse (3) ein kompaktes Luftionisationsmodul sind.

10. Luftionisationsmodul nach Patentanspruch 1, **dadurch gekennzeichnet, dass** der Körper (6) aus dem wärmeisolierenden Material zwischen der Montageplatte (4) und der Leiterplatte (5) aus einem geschäumten Kunststoff besteht.

11. Luftionisationsmodul nach Patentanspruch 1 oder 6, **dadurch gekennzeichnet, dass** vier Ionisationsröhren (2) eine Gruppe von Ionisationsröhren (2) sind und dass die Ionisationsröhren (2) an den Eckpunkten eines keinen rechten Winkel aufweisenden Parallelogramms angeordnet sind.

## Claims

1. An air ionization module with ionization tubes (2) that are detachably arranged in sockets and a carrier (1) with the sockets, **characterized in that** the carrier (1) features two spaced-apart boards (4, 5), wherein a first board (4) is a mounting board (4) and a second board (5) is a printed circuit board (5) with the sockets, and **in that** a body (6) of a thermally insulating material, preferably a foamed plastic, is arranged between the boards (4, 5) such that the second board (5) is thermally insulated relative to the first board (4).

2. The air ionization module according to claim 1, **characterized in that** the body (6) of a thermally insulating material is arranged between the boards (4, 5) and a wall (7) enclosing this body (6), wherein said wall consists of a material that is thermally insulating at least in certain areas.

3. The air ionization module according to claim 1, **characterized in that** the boards (4, 5) are connected to one another by means of snap-in mechanisms, wherein snap-in elements engage into recesses.

4. The air ionization module according to claim 1, **characterized in that** the mounting board (4) is larger than the printed circuit board (5).

5. The air ionization module according to claim 1, **characterized in that** the sockets are arranged in the body (6) of a thermally insulating material such that they end flush with the outwardly directed surface of the printed circuit board (5).

6. The air ionization module according to claim 1 or 5, **characterized in that** the sockets are electrically connected to strip conductors of the printed circuit board (5) and thereby simultaneously mounted, **in that** anodes of the ionization tubes (2) are connected to one another by means of sockets and strip conductors of the printed circuit board (5), **in that** at least one component of an electrical connecting element for a detachably connection of at least one cathode is electrically connected to at least one strip conductor of the printed circuit board (5) and thereby simultaneously mounted, and **in that** the interconnected anodes and cathodes of ionization tubes (2) form a group of ionization tubes (2).

7. The air ionization module according to claim 1 or 6, **characterized in that** the anodes of the ionization tubes (2) of a group are interconnected to the secondary winding of a transformer (8), **in that** the primary winding of the transformer (8) can be connected to an electric low-voltage network (10) by means of a switch (9), and **in that** the cathodes of the ionization tubes (2) are grounded.

8. The air ionization module according to claim 7, **characterized in that** the actuating device of a mechanical or the control electrode of an electrical switch (9) is connected to a data processing system such that an automatic and/or predefined operation of the ionization tubes (2) can be realized by means of the data processing system.

9. The air ionization module according to claim 1, 6 or 7, **characterized in that** transformers (8) for groups of ionization tubes (2) are arranged in a housing (3), and **in that** the housing (3) is mounted on the mounting board (4) such that the ionization tubes (2), the carrier (1) and the housing (3) form a compact air ionization module.

10. The air ionization module according to claim 1, **characterized in that** the body (6) of a thermally insulating material between the mounting board (4) and the printed circuit board (5) consists of a foamed plastic.

11. The air ionization module according to claim 1 or 6, **characterized in that** four ionization tubes (2) form a group of ionization tubes (2), and **in that** the ionization tubes (2) are arranged on the corners of a parallelogram that has no right angle.

## Revendications

1. Module d'ionisation de l'air avec des tubes d'ionisation (2) disposés de manière amovible dans des montures et un support (1) avec les montures, **caractérisé en ce que** le support (1) comporte deux plaques (4, 5) disposées éloignées l'une de l'autre, une première plaque (4) étant une plaque de montage (4) et une deuxième plaque (5) étant une plaque de circuits imprimés (5) avec des montures et **en ce qu'**un corps (6) en matériau thermo-isolant, de préférence en mousse de matière plastique, se trouve entre les plaques (4, 5) de telle manière que la deuxième plaque (5) est disposée de manière thermo-isolante en face de la première plaque (4).

2. Module d'ionisation de l'air selon la revendication 1, **caractérisé en ce que** le corps (6) en matière thermo-isolante se trouve entre les plaques (4, 5) et une paroi (7) en un matériau au moins en partie thermo-isolant entourant ce corps (6).

3. Module d'ionisation de l'air selon la revendication 1, **caractérisé en ce que** les plaques (4, 5) sont reliées l'une à l'autre par des mécanismes d'encliquetage, des éléments d'encliquetage venant en prise dans des cavités.

4. Module d'ionisation de l'air selon la revendication 1, **caractérisé en ce que** la plaque de montage (4) est plus grande que la plaque de circuits imprimés (5).

5. Module d'ionisation de l'air selon la revendication 1, **caractérisé en ce que** les montures se trouvent dans le corps (6) en matériau thermo-isolant de telle manière que celles-ci viennent affleurer la surface tournée vers l'extérieur de la plaque de circuits imprimés (5).

6. Module d'ionisation de l'air selon la revendication 1 ou 5, **caractérisé en ce que** les montures sont électriquement reliées aux réseaux de circuits imprimés de la plaque de circuits imprimés (5) et sont ainsi en même temps fixées de sorte que les anodes des tubes d'ionisation (2) sont reliées entre elles par les montures et les réseaux de circuits imprimés de la plaque de circuits imprimés (5), **en ce qu'**au moins un composant d'un élément de liaison électrique pour une liaison amovible d'au moins une cathode est relié de manière électro-conductrice à au moins un réseau de circuits imprimés de la plaque de circuits imprimés (5) et est de ce fait en même temps fixé et **en ce que** les anodes et cathodes reliées entre elles des tubes d'ionisation (2) sont un groupe de tubes d'ionisation (2).

7. Module d'ionisation de l'air selon la revendication 1 ou 6, **caractérisé en ce que** les anodes des tubes d'ionisation (2) d'un groupe sont interconnectées à un enroulement secondaire d'un transformateur (8), **en ce que** l'enroulement primaire du transformateur (8) peut être relié par un contacteur (9) à un réseau basse tension électrique (10) et **en ce que** les cathodes des tubes d'ionisation (2) sont mises à la terre.

8. Module d'ionisation de l'air selon la revendication 7, **caractérisé en ce que** le dispositif de commande d'un contacteur à action mécanique ou l'électrode de commande d'un contacteur (9) pouvant être commandé électriquement est relié à un système de traitement des données de telle manière qu'un fonctionnement automatique et/ou prédéfini des tubes d'ionisation (2) est assuré au moyen du système de traitement des données.

9. Module d'ionisation de l'air selon la revendication 1, 6 ou 7, **caractérisé en ce que** les transformateurs (8) pour des groupes de tubes d'ionisation (2) sont disposés dans un boîtier (3) et **en ce que** le boîtier (3) est fixé à la plaque de montage (4) de telle manière que les tubes d'ionisation (2), le support (1) et le boîtier (3) constituent un module d'ionisation de l'air compact.

10. Module d'ionisation de l'air selon la revendication 1, **caractérisé en ce que** le corps (6) en matériau thermo-isolant entre la plaque de montage (4) et la plaque de circuits imprimés (5) est en mousse de matière plastique.

11. Module d'ionisation de l'air selon la revendication 1 ou 6, **caractérisé en ce que** les quatre tubes d'ionisation (2) forment un groupe de tubes d'ionisation (2) et **en ce que** les tubes d'ionisation (2) sont disposés aux points d'angle d'un parallélogramme ne comportant aucun angle droit.
